# EUROPEAN PATENT SPECIFICATION

(11) **EP 2 868 208 B1**
(45) Date of publication and mention of the grant of the patent: **17.04.2019**
(21) Application number: 13191063.0
(22) Date of filing: 31.10.2013
(51) Int. Cl.: A23L 2/39, A23L 2/84, A23L 33/15, C12G 3/08, A61K 31/375

(54) **Method for the preparation of a low-hygroscopic, dried extract of a vitamin C rich fruit or vegetable species**
Verfahren zur Herstellung eines trockenen Extrakts mit geringem Feuchtigkeitsgehalt einer an Vitamin C reichen Obst- oder Gemüsesorte
Procédé pour la préparation d'un extrait sec faiblement hygroscopique d'une vitamine C riche d'espèces de fruits ou de légumes

(43) Date of publication of application: 06.05.2015
(73) Proprietor: Plantextrakt GmbH & Co. KG, 91487 Vestenbergsgreuth (DE)
(72) Inventor: Bonnländer, Bernd, 90571 Schwaig (DE); Kriesl, Erwin, 90579 Langenzenn (DE)
(74) Representative: Hübner, Gerd

(56) References cited:
- WO-A1-01/03520
- WO-A1-89/07132
- WO-A2-2007/141420
- CH-A5- 632 137
- JP-A- 2005 154 432
- US-A- 3 012 942
- US-A- 3 086 915
- DANIELA FRACASSETTI ET AL: "Ellagic acid derivatives, ellagitannins, proanthocyanidins and other phenolics, vitamin C and antioxidant capacity of two powder products from camu-camu fruit (Myrciaria dubia)", FOOD CHEMISTRY, vol. 139, no. 1-4, 1 August 2013 (2013-08-01), pages 578-588, XP055116812, ISSN: 0308-8146, DOI: 10.1016/j.foodchem.2013.01.121
- MOREIRA G E G ET AL: "Physical properties of spray dried acerola pomace extract as affected by temperature and drying aids", LWT- FOOD SCIENCE AND TECHNOLOGY, ACADEMIC PRESS, UNITED KINGDOM, vol. 42, no. 2, 1 March 2009 (2009-03-01), pages 641-645, XP025656567, ISSN: 0023-6438, DOI: 10.1016/J.LWT.2008.07.008 [retrieved on 2008-07-24]
- NANTZ M P ET AL: "Immunity and antioxidant capacity in humans is enhanced by consumption of a dried, encapsulated fruit and vegetable juice concentrate", THE JOURNAL OF NUTRITION, AMERICAN SOCIETY FOR NUTRITION, US, vol. 136, no. 10, 1 January 2006 (2006-01-01), pages 2606-2610, XP002581661, ISSN: 0022-3166 [retrieved on 2006-03-09]

## Description

The present invention refers to a process for the preparation of a low-hygroscopic, dried extract of a vitamin C rich fruit or vegetable species, i.e. a natural product rich in vitamin C of only natural origin from fruits or vegetable in stable, dried form.

The background of the invention is to be commented as follows. As concerns food products the market and customers nowadays prefer natural origin substances over chemically synthesized compounds. This requires industrial, economic processes to enrich and stabilize substances from natural sources.

Acerola (Malpighia glabra) is a tropical plant producing fruits rich in vitamin C. Also Camu Camu (Myrciaria dubia) is an Amazonian tree producing fruits very rich in vitamin C. Other fruits and vegetables which are rich in vitamin C are rose hip, bell pepper, sea buckthorn, orange, black currant or guava.

The fruits, juices and juice concentrates of these plants are commercialized, but have limitations because of their water content and thus low stability as well as ineffective shipping. For these reasons drying of such products is performed by known processes to the experts such as spray, freeze or belt drying. However, these techniques are limited in the case of the described products, mainly because of the high sugar content leading to melting and/or highly hygroscopic products, which are not feasible for food or pharma industry.
Classical drying aids like maltodextrines or other carriers do not solve the problem, as the relative content of Vitamin C is reduced if these carriers are added.

JP 2005 154432 A discloses the fermentation of pre-concentrated acerola juice with S. cerevisiae and subsequent freeze drying of the product. The fermentation parameters are 20 hours at 32 °C.

US 3,012,942 A and US 3,086,915 relate to the production of sugar-free dry concentrates of acerola juice wherein the sugar content is removed by fermentation. The fermentation parameters are in the order of less than a day to more than 2 days and temperatures at e.g. 26 °C.

Now the object of the present invention is to provide a method for the preparation of a low-hygroscopic, dried powder extract of vitamin C rich fruit or vegetable species which method is easy and effective to be performed on an industrial scale and leads to a product which is improved as concerns its hygroscopicity.

This object is achieved by the method according to the steps of claim 1 which are:
- preparing a juice of the species,
- removing at least a part of the sugars contained in the juice by a fermentative process not altering other ingredients in the juice, wherein the fermentation time is between 2 and 6 days, preferably between 2 and 4 days and the fermentation temperature is between 10°C and 50°C, preferably between 20°C and 30°C,
- removing the ethanol content from the juice by treating the residue including the vitamin C contents to get a vitamin C rich wet extract as residue including the vitamin C contents, wherein as characterising feature the removal of the ethanol takes place by a UHT process, preferably a direct steam treatment UHT process, and
- drying the residue to a low hygroscopic powder.

Due to the use of processes to reduce or remove the sugar content in such products by fermentation as is further claimed in the depending claims, the remaining compounds including vitamin C (ascorbic acid) in the fruit or vegetable bases are enhanced linked to the reduction of sugar in the final product.

The biochemical method to remove the sugar contents from the juice is based on a fermentation by adding yeast, preferably of the species Saccharomyces cerevisiae, to the juice. Thus the sugar contents is fermented to ethanol whereafter the ethanol is removed to get the vitamin C rich wet extract as an intermediate product of the process of the present invention.

Within this alternative embodiment preferably a concentrated juice is used which is diluted to a solution containing 10 % to 30 %, preferably 20 % of soluble solids. All aforesaid parameters help to preserve the high contents of vitamin C on the one hand and to optimise the yield of the production process according to the invention on the other hand.

To remove yeast the fermented solution is centrifuged or filtrated and afterwards as an option being evaporated to a concentration higher than 50 % soluble solids by a process with low thermal input, preferably under vacuum.

Finally the vitamin C rich wet extract may be dried to the low hygroscopic powder by spray drying, freeze drying or any other appropriate drying technique.

Summing up the present invention describes a process to remove said sugars in such products by fermentation processes, not altering significantly other ingredients in the fruit or vegetable products. So a dried, low-hygroscopic product can be produced offering the enhanced stability of such dry products but having high content of ascorbic acid in such products.

To further facilitate such process the starting material can be of such maturity that the relative content of ascorbic acid is the highest, in case of fruits that is mainly unripe fruits (Weber et al. "Metabolic and isotopic correlations between D-Glucose, L-Ascorbic acid and L-Tartaric Acid", Isotopes Environ. Health Stud. 1997, 33, 151-155).

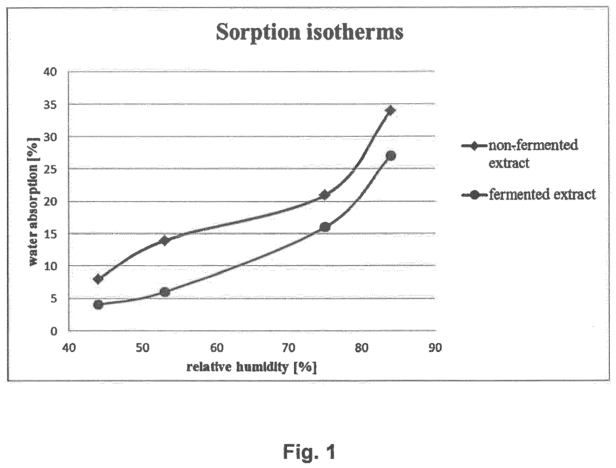

Further aspects and advantages of the invention can be taken by the following description of preferred embodiments. The only drawing in
- **Fig. 1**: shows a diagram of sorption isotherms of a non-fermented and a fermented extract

### Example 1 (for illustrative purposes only):

1 kg of concentrated Acerola fruit juice (28 °Brix) is diluted to 10 % soluble solids. Yeast, e.g. of the species Saccharomyces cerevisiae, is added at temperatures around its optimal working conditions depending on the type of yeast used. The parameters are taken from a temperature range of preferably 20-25°C and a storage time of usually 3 days. After analysis of the remaining sugar content in the reaction mixture by an enzymatic test the fermented solution is filtrated or separated.

The intermediate product thus obtained is a vitamin C rich wet extract which is concentrated by a rising film plate evaporator as state of the art concentration technique with low thermal input, preferably under vacuum to maintain the ascorbic acid. At a concentration usually higher than 50 % soluble solids such extract can be used as it is or dried by techniques known to the experts such as spray drying, freeze drying or other drying techniques and produces a low-hygroscopic powder.

The mentioned enrichment of Vitamin C becomes clear from the following table 1, in which the non-fermented extract is a spray dried Acerola juice concentrate, which has not been treated with yeast and the fermented extract is that of example 1. Both extracts contain 60 % maltodextrin as spray drying agent.

**Table 1: sugar and vitamin C content of fermented and non-fermented extract**

| | Non-fermented extract | Fermented extract |
|---|---|---|
| | Content [%] | |
| Vitamin C | 13.33 | 18.98 |
| Fructose | 4.29 | 0.03 |
| Glucose | 3.25 | 0.09 |
| Saccharose | 0.01 | 0.01 |

### Example 2:

1 kg of concentrated organic Acerola fruit juice (50 °Brix) is diluted to 20 % soluble solids. Yeast, e.g. of the species Saccharomyces cerevisiae, is added at temperatures around its optimal working conditions depending on the type of yeast used. The parameters are taken from a temperature range of preferably 20-25°C and a storage time of usually 3 days. After analysis of the remaining sugar content by HPLC or enzymatic tests the fermented solution is separated.

The intermediate product thus obtained is a vitamin C rich wet extract which is UHT treated, preferably a direct steam treatment UHT process, to remove ethanol. The resulting extract can be dried by techniques as described in example 1.

### Product check:

To prove the low-hygroscopic properties the extracts obtained as explained above are stored in water saturated atmosphere with different relative humidity. The different relative humidities were adjusted by the saturated salt solutions as they are shown in the following table 2:

**Table 2: saturated salt solution for adjusting relative humidity**

| **Salt** | **Relative humidity [%]** |
|---|---|
| Sodium carbonate | 44 |
| Magnesium carbonate | 53 |
| Sodium chloride | 75 |
| Potassium chloride | 84 |

These products obtained by the processes according to the invention do not show any physical changes up to 53 % relative humidity in the powder form. Compared to common extracts containing the same carrier content the extract according to the invention behaves much less hygroscopic, what is shown in Fig. 1 presenting the diagram of according sorption isotherms of the non-fermented and fermented extracts according to table 1. The water absorption of a fermented extract is considerably lower - between 4 and 8 percentage points - throughout the complete range between 40% and 85% of practical values of the relative humidity as compared to a non-fermented extract.

## Claims

1. Method for the preparation of a low-hygroscopic, dried extract of a vitamin C rich fruit or vegetable species, like Acerola (Malpighia glabra) or Camu Camu (Myrciaria dubia), including the steps of
- preparing a juice of the species,
- removing at least a part of the sugars contained in the juice by a fermentative process generating an ethanol content in the juice but not altering other ingredients in the juice, wherein the fermentation time is between 2 and 6 days, preferably between 2 and 4 days and the fermentation temperature is between 10°C and 50°C, preferably between 20°C and 30°C,
- removing the ethanol content from the juice by treating the residue including the vitamin C contents to get a vitamin C rich wet extract as residue including the vitamin C contents, and
- drying the residue to a low-hygroscopic powder,
**characterized in that** the ethanol is removed by a UHT process, preferably a direct steam treatment UHT process.

2. Method according to claim 1, **characterized in that** for removing the sugar by a biochemical process a yeast is added to the juice to ferment the sugar to ethanol.

3. Method according to claim 2, **characterized in that** as juice a concentrated juice is used which is diluted to a solution containing 10 % to 30 %, preferably 20 % of soluble solids.

4. Method according to claim 2 or 3, **characterized in that** a yeast of the species Saccharomyces cerevisiae is used.

5. Method according to one of the preceding claims, **characterized in that** the vitamin C rich wet extract is concentrated to a concentration higher than 50 % soluble solids by a process with low thermal input, preferably under vacuum.

6. Method according to one of the preceding claims, **characterized in that** the vitamin C rich wet extract is dried to the low hygroscopic powder by spray drying or freeze drying.

## Patentansprüche

1. Verfahren zur Herstellung eines getrockneten Extrakts mit geringem Feuchtigkeitsgehalt aus einer an Vitamin C reichen Obst- oder Gemüsesorte, wie z.B. Acerola (Malpighia glabra) oder Camu Camu (Myrciaria dubia), umfassend die folgenden Schritte:
- Herstellen eines Safts aus der Sorte,
- Entfernen zumindest eines Teils der in dem Saft enthaltenen Zucker durch einen Fermentationsvorgang, wodurch ein EthanolGehalt in dem Saft erzeugt wird, andere Inhaltsstoffe in dem Saft jedoch nicht verändert werden, wobei die Fermentationszeit zwischen 2 und 6 Tagen, vorzugsweise zwischen 2 und 4 Tagen beträgt und die Fermentationstemperatur zwischen 10°C und 50°C, vorzugsweise zwischen 20°C und 30°C liegt,
- Entfernen des Ethanol-Gehalts aus dem Saft durch Behandeln des Rückstandes umfassend den Vitamin-C-Gehalt, so dass ein an Vitamin C reicher Extrakt mit hohem Feuchtigkeitsgehalt als Rückstand umfassend den Vitamin-C-Gehalt entsteht, und
- Trocknen des Rückstandes zu einem Pulver mit geringem Feuchtigkeitsgehalt,
**dadurch gekennzeichnet, dass** das Ethanol in einem UHT-Verfahren, vorzugsweise einem Direktdampfbehandlungs-UHT-Verfahren, entfernt wird.

2. Verfahren gemäß Anspruch 1, **dadurch gekennzeichnet, dass** dem Saft ein Hefepilz zugegeben wird, um den Zucker durch ein biochemisches Verfahren zu entfernen, in dem der Zucker zu Ethanol fermentiert wird.

3. Verfahren gemäß Anspruch 2, **dadurch gekennzeichnet, dass** ein konzentrierter Saft als Saft verwendet wird, der zu einer Lösung enthaltend 10% bis 30%, vorzugsweise 20%, an löslichen Feststoffen verdünnt wird.

4. Verfahren gemäß Anspruch 2 oder 3, **dadurch gekennzeichnet, dass** ein Hefepilz der Gattung Saccharomyces cerevisiae verwendet wird.

5. Vefahren gemäß einem der vorigen Ansprüche, **dadurch gekennzeichnet, dass** der an Vitamin C reiche Extrakt mit hohem Feuchtigkeitsgehalt in einem Verfahren mit niedrigem Wärmeeintrag, vorzugsweise unter Vakuum, zu einem Konzentrat mit mehr als 50% an löslichen Feststoffen konzentriert wird.

6. Verfahren gemäß einem der vorigen Ansprüche, **dadurch gekennzeichnet, dass** der an Vitamin C reiche Extrakt mit hohem Feuchtigkeitsgehalt durch Sprühtrocknung oder Gefriertrocknung zu dem Pulver mit geringem Feuchtigkeitsgehalt getrocknet wird.

## Revendications

1. Procédé pour la préparation d'un extrait séché faiblement hygroscopique d'une espèce de fruit ou de légume riche en vitamine C, comme l'acérola (Malphighia glabra) ou le camu-camu (Myrciaria dubia), comprenant les étapes suivantes :
- préparation d'un jus de l'espèce,
- retrait d'au moins une partie des sucres contenus dans le jus par un traitement de fermentation générant une teneur en éthanol dans le jus mais n'altérant pas d'autres ingrédients dans le jus, où le temps de fermentation est compris entre 2 et 6 jours, de préférence entre 2 et 4 jours, et la température de fermentation est comprise entre 10 °C et 50 °C, de préférence entre 20 °C et 30 °C,
- retrait de la teneur en éthanol du jus par traitement du résidu, y compris la teneur en vitamine C, pour que soit obtenu un extrait humide riche en vitamine C sous la forme d'un résidu contenant la teneur en vitamine C, et
- séchage du résidu en une poudre faiblement hygroscopique,
**caractérisé en ce que** l'éthanol est retiré par un traitement UHT, de préférence un traitement UHT à la vapeur directe.

2. Procédé selon la revendication 1, **caractérisé en ce que**, pour retirer le sucre par un traitement biochimique, une levure est ajoutée au jus pour fermenter le sucre en éthanol.

3. Procédé selon la revendication 2, **caractérisé en ce que**, en tant que jus, est utilisé un jus concentré qui est dilué jusqu'à une solution contenant 10 % à 30 %, de préférence 20 % de solides solubles.

4. Procédé selon la revendication 2 ou 3, **caractérisé en ce qu'**une levure de l'espèce Saccharomyces cerevisiae est utilisée.

5. Procédé selon l'une quelconque des revendications précédentes, **caractérisé en ce que** l'extrait humide riche en vitamine C est concentré à une concentration supérieure à 50 % de solides solubles par un traitement avec un faible apport thermique, de préférence sous vide.

6. Procédé selon l'une quelconque des revendications précédentes, **caractérisé en ce que** l'extrait humide riche en vitamine C est séché en la poudre faiblement hygroscopique par séchage par pulvérisation ou par lyophilisation.
